# Europäisches Patentamt

# European Patent Office

# Office européen des brevets

(11) Publication number: **0 073 646**
**B1**

# EUROPEAN PATENT SPECIFICATION

(45) Date of publication of patent specification: **17.05.89**

(21) Application number: **82304478.9**

(22) Date of filing: **25.08.82**

(51) Int. Cl.⁴: **C 12 N 15/00**, C 07 H 21/04,
C 12 N 1/00, A 61 K 37/02 //
C12R1/19

(54) **Construction of DNA sequences and their use for microbial production of proteins, in particular human serum albumin.**

(30) Priority: **28.08.81 US 297380**

(43) Date of publication of application:
**09.03.83 Bulletin 83/10**

(45) Publication of the grant of the patent:
**17.05.89 Bulletin 89/20**

(84) Designated Contracting States:
**AT BE CH DE FR GB IT LI LU NL SE**

(56) References cited:
EP-A-0 065 924
EP-A-0 079 739
EP-A-0 091 527
WO-A-82/03632

NATURE, vol. 287, no. 5779, 18th September
1980, Basingstoke, GB, R. DERYNCK et al.:
"Expression of human fibroblast interferon
gene in Escherichia coli", pp. 193-197

(73) Proprietor: **GENENTECH, INC.**
**460 Point San Bruno Boulevard**
**South San Francisco California 94080 (US)**

(72) Inventor: **Lawn, Richard M.**
**1927 8th Avenue**
**San Francisco California 94116 (US)**

(74) Representative: **Armitage, Ian Michael et al**
**MEWBURN ELLIS & CO. 2/3 Cursitor Street**
**London EC4A 1BQ (GB)**

(56) References cited:
NATURE, vol. 285, 19th June 1980, Macmillan
Journals Ltd., R. DERYNCK et al.: "Isolation and
structure of a human fibroplast interferon
gene", pp. 542-546

Courier Press, Leamington Spa, England.

(56) References cited:

CELL, vol. 18, October 1979, B. CORDELL et al.: "Isolation and characterization of a cloned rat insulin gene", pp. 533-543

NATURE, vol. 287, no. 5781, 2nd October 1980, Chesham Bucks, GB, D.V. GOEDDEL et al.: "Human leukocyte interferon produced by E.coli is biologically active", pp. 411-415

NUCLEIC ACIDS RESEARCH, vol. 9, no. 22, 21th May 1981, IRL Press Ltd., London, GB, R.M. LAWN et al.: "The sequence of human serum albumin cDNA and its expression in E.coli"

THE MERCK INDEX, 9th ed., Merck & Co. Ltd. 1976, ref. 8212, New York, US, "Serum Albumin"

NATURE, vol. 291, 21th May 1981, Macmillan Journals Ltd., S.W. LAW et al.: "Homology between the primary structure of alfa-fetoprotein, deduced from a complete cDNA sequence, and serum albumin", pp. 201-205

PROC. NATL. ACAD. SCI. vol. 78, no. 1, January 1981, Biochemistry T.D. SARGENT et al.: "Nucleotide sequence of cloned rat serum albumin messenger RNA", pp. 243-241

THE JOURNAL OF CELL BIOLOGY, vol. 87, no. 2, part 2, P111A, ref. D 832, J.W. HAWKINS et al.: "Isolation and characterization of the natural gene for human serum albumin"

BIOSCIENCES INFORMATION SERVICE, Philadelphia, US, Biological Abstracts/RRM 1981, ref. 23066348, A. DUGAICZYK et al.: "DNA sequence analysis of alpha feto protein and serum albumin"

## Description

This invention relates to recombinant DNA technology. It particularly relates to the application of the technology to the production of human serum albumin (HSA) in microorganisms for use in the therapeutic treatment of humans. In one aspect the invention relates to a technique for producing DNA sequences encoding desired polypeptides. In another aspect it relates to the construction of microbial expression vehicles containing DNA sequences encoding a protein, e.g. human serum albumin or the biologically active component thereof operably linked to expression effecting promoter systems and to the expression vehicles so constructed. In another aspect, the present invention relates to microorganisms transformed with such expression vehicles, thus directed in the expression of the DNA sequences referred to above. In yet other aspects, this invention relates to the means and methods of converting the end product of such expression to entities, such as pharmaceutical compositions, useful for the therapeutic treatment of humans. In preferred embodiments, this invention provides for particular expression vectors that are sequenced properly such that mature human serum albumin is produced directly.

In one aspect, the present invention is particularly directed to a method of preparing cDNA encoding polypeptides or biologically active portions thereof. This aspect provides the means and methods of utilizing synthetic primer DNA corresponding to a portion of the mRNA of the intended polypeptide, adjacent to a known endonuclease restriction site, in order to obtain by reverse transcription a series of DNA fragments encoding sequences of the polypeptide. These fragments are prepared such that the entire desired protein coding sequence is represented, the individual fragments containing overlapping DNA sequences harboring common endonuclease restriction sites within the corresponding overlapping sequence. This aspect facilitates the selective cleavage and ligation of the respective fragments so as to assemble the entire cDNA sequence encoding the polypeptide in proper reading frame. This discovery permits the obtention of cDNA of high molecular weight proteins which otherwise may not be available through use of usual reverse transcriptase methods and/or chemical synthesis.

The publications and other materials hereof used to illuminate the background of the invention, and in particular cases, to provide additional details respecting its practice are incorporated herein by reference, and for convenience, are numerically referenced in the following text and grouped in the appended bibliography.

(A) Human serum albumin

Human erum albumin (HSA) is the major protein species in adult serum. It is produced in the liver and is largely responsible for maintaining normal osmolarity in the bloodstream and functions as a carrier for numerous serum molecules (1, 2). The apparent fetal counterpart of HSA is α-fetoprotein and studies have been undertaken to compare the two as well as rat serum albumin and α-fetoprotein (3—8). The complete protein sequence of HSA has been published (9—12). The published protein sequences of HSA disagree in about 20 residues as well as in the total number of amino acids in the mature protein [584 amino acids (9); 585 (12)]. Some evidence suggests that HSA is initially synthesized as a precursor molecule (13, 14) containing a "prepro" sequence. The precursor forms of bovine (15) and rat (16) serum albumin have also been sequenced.

The role or rationale for the use of albumin in therapeutic application is for the treatment of hypovolemia, hypoproteinemia and shock. Albumin currently is used to improve the plasma oncotic (colloid osmotic) pressure, caused by solutes (colloids) which are not able to pass through capillary pores. Inasmuch as albumin has a low permeability constant, it essentially confines itself to the intravascular compartment. When different concentrations of nondiffusable particles exist on opposite sides of the cell membrane, water crosses the partition until the concentrations of particles are equal on both sides. In this process of osmosis, albumin plays a vital role in maintaining the liquid content in blood.

Thus, the therapeutic benefits of albumin administration reside primarily for the treatment of conditions where there is a loss of liquid from the intravascular compartment, such as in surgical operations, shock, burns, and hypoproteinemia resulting in edema. Albumin is also used for diagnostic applications in which its nonspecific ability to bind to other proteins makes it useful in various diagnostic solutions.

Presently, human serum albumin is produced from whole blood fractionation techniques, and thus is not available in large amounts at competitive costs. The application of recombinant DNA technology makes possible the production of copious amounts of human serum albumin by use of genetically directing microorganisms to produce it efficiently. The present invention may enable the availability of purified HSA produced through recombinant DNA technology more abundantly and at lower cost than is now presently possible. The present invention also provides knowledge of the DNA sequence organization of human serum albumin and its deduced amino acid sequence, helping to elucidate the evolutionary, regulatory, and functional properties of human serum albumin as well as its related proteins such as alpha-fetoprotein.

More particularly, present invention provides for the isolation of cDNA clones spanning the entire sequence of the protein coding and 3' untranslated portions of HSA mRNA. These cDNA clones were used to construct a recombinant expression vehicle which directed the expression in a microorganism strain of the mature HSA protein under control of the trp promoter. The present invention also provides the complete nucleotide and deduced amino acid sequence of HSA.

Reference herein to the expression of "mature human serum albumin" connotes the microbial production of human serum albumin unaccompanied by the presequence ("prepro") that immediately attends translation of the human serum albumin mRNA. Mature human serum albumin, according to the present invention, is immediately expressed from a translation start signal (ATG), which also encodes the amino acid methionine linked to the first amino acid of albumin. This methionine amino acid can be naturally cleaved by the microorganism so as to prepare the human serum albumin directly. Mature human serum albumin can be expressed together with a conjugated protein other than the conventional leader, the conjugate being specifically cleavable in an intra- or extracellular environment. See British patent publication number 2007676A. Finally, the mature human serum albumin can be produced in conjunction with a microbial signal polypeptide which transports the conjugate to the cell wall, where the signal is processed away and the mature human serum albumin secreted.

(B) Recombinant DNA technology

With the advent of recombinant DNA technology, the controlled microbial production of an enormous variety of useful polypeptides has become possible. Many mammalian polypeptides, such as human growth hormone and human and hybrid leukocyte interferons, have already been produced by various microorganisms. The power of the technology admits the microbial production of an enormous variety of useful polypeptides, putting within reach the microbially directed manufacture of hormones, enzymes, antibodies, and vaccines useful for a variety of drug-targeting applications.

A basic element of recombinant DNA technology is the plasmid, an extrachromosomal loop of double-stranded DNA found in bacteria oftentimes in multiple copies per cell. Included in the information encoded in the plasmid DNA is that required to reproduce the plasmid in daughter cells (i.e., a "replicon" or origin of replication) and ordinarily, one or more phenotypic selection characteristics, such as resistance to antibiotics, which permit clones of the host cell containing the plasmid of interest to be recognized and preferentially grown in selective media. The utility of bacterial plasmids lies in the fact that they can be specifically cleaved by one or another restriction endonuclease or "restriction enzyme", each of which recognizes a different site on the plasmid DNA. Thereafter heterologous genes or gene fragments may be inserted into the plasmid by endwise joining at the cleavage site or at reconstructed ends adjacent to the cleavage site. (As used herein, the term "heterologous" refers to a gene not ordinarily found in, or a polypeptide sequence ordinarily not produced by, a given microorganism, whereas the term "homologous" refers to a gene or polypeptide which is found in, or produced by the corresponding wild-type microorganism.) Thus formed are so-called replicable expression vehicles.

DNA recombination is performed outside the microorganism, and the resulting "recombinant" replicable expression vehicle, or plasmid, can be introduced into microorganisms by a process known as transformation and large quantities of the heterologous gene-containing recombinant vehicle obtained by growing the transformant. Moreover, where the gene is properly inserted with reference to portions of the plasmid which govern the transcription and translation of the encoded DNA message, the resulting expression vehicle can be used to actually produce the polypeptide sequence for which the inserted gene codes, a process referred to as expression.

Expression is initiated in a DNA region known as the promoter. In the transcription phase of expression, the DNA unwinds, exposing the sense coding strand of the DNA as a template for initiated synthesis of messenger RNA from the 5' to 3' end of the entire DNA sequence. The messenger RNA is, in turn, bound by ribosomes, where the messenger RNA is translated into a polypeptide chain having the amino acid sequence for which the DNA codes. Each amino acid is encoded by a nucleotide triplet or "codon" which collectively make up the "structural gene", i.e., that part of the DNA sequence which encodes the amino acid sequence of the expressed polypeptide product.

Translation is initiated at a "start" signal (ordinarily ATG, which in the resulting messenger RNA becomes AUG). So-called stop codons, transcribed at the end of the structural gene, signal the end of translation and, hence, the production of further amino acid units. The resulting product may be obtained by lysing the host cell and recovering the product by appropriate purification from other proteins.

In practice, the use of recombinant DNA technology can express entirely heterologous polypeptides—so-called direct expression—or alternatively may express a heterologous polypeptide, fused to a portion of the amino acid sequence of a homologous polypeptide. In the latter cases, the intended bioactive product is rendered bioinactive within the fused, homologous/heterologous polypeptide until it is cleaved in an extracellular environment. See Wetzel, American Scientist 68, 664 (1980).

If recombinant DNA technology is to fully sustain its promise, systems must be devised which optimize expression of gene inserts, so that the intended polypeptide products can be made available in controlled environments and in high yields.

(C) State of the art

Sargent *et al.*, in *Proc. Natl. Acad. Sci, (USA) 78*, 243 (1981), describe the cloning of rat serum albumin messenger RNA in a series of recombinant DNA plasmids. This was done to determine the nucleotide sequences of the clones in order to study the evolutionary hypothesis of the protein product. Thus, these workers made no attempt to assemble the cDNA fragments they prepared.

In *Journal of Supramolecular Structure and Cellular Biochemistry.* Supplement 5, 1981, Alan R. Liss, Inc. NY, Dugaiczyk *et al.* report, in abstract form, their studies of the human gene for human serum albumin. They obtained cDNA fragments but there is no evidence that these workers cloned or produced by fragments for any purpose other than for studying the basic molecular biology of the α-fetoprotein and serum albumin genes.

The present invention is based upon the discovery that recombinant DNA technology can be used to successfully and efficiently produce human serum albumin in direct form. The product is suitable for use in therapeutic treatment of human beings in need of supplementation of albumin. The product is produced by genetically directed microorganisms and thus the potential exists to prepare and isolate HSA in a more efficient manner that is presently possible by blood fractionation techniques. It is noteworthy that we have succeeded in genetically directing a microorganism to produce a protein of enormous length—584 amino acids corresponding to an mRNA transcript upwards of about 2,000 bases.

The present invention comprises the human serum albumin thus produced and the means and methods of its production. The present invention is further directed to replicable DNA expression vehicles harboring gene sequences encoding HSA in directly expressible form. Further, the present invention is directed to microorganism strains transformed with the expression vehicles described above and to microbial cultures of such transformed strains, capable of producing HSA. In still further aspects, the present invention is directed to various processes useful for preparing said HSA gene sequences, DNA expression vehicles, microorganism strains and cultures and to specific embodiments thereof. Still further, this invention is directed to the preparation of cDNA sequences encoding polypeptides which are heterologous to the microorganism host, such as human serum albumin, utilizing synthetic DNA primer sequences corresponding in sequence to regions adjacent to known restriction endonuclease sites, such that individual fragments of cDNA can be prepared which overlap in the regions encoding the common restriction endonuclease sites. This embodiment enables the precise cleavage and ligation of the fragments so as to prepare the properly encoded DNA sequence for the intended polypeptide.

The work described herein involved the expression of human serum albumin (HSA) as a representative polypeptide which is heterologous to the microorganism employed as host. Likewise the work described involved use of the microorganism *E. coli* K-12 strain 294 (end A, thi⁻, hsr⁻, ₖhsm⁺), as described in British Patent Publication No. 2055382A. This strain has been deposited with the American Type Culture Collection, ATCC Accession No. 31446.

The invention, in its most preferred embodiments, is described with reference to *E. coli*, including not only strain *E. coli* K-12 strain 294, defined above, but also other known *E. coli* strains such as *E. coli* B, *E. coli* X 1776 and *E. coli* W 3110, or other microbial strains many of which are deposited and (potentially) available from recognized microorganism depository institutions, such as the American Type Culture Collection (ATCC)—cf. the ATCC catalogue listing. Seel also German Offenlegungsschrift 2644432. These other microorganisms include, for example, Bacilli such as *Bacillus subtilis* and other enterobacteriaceae among which can be mentioned as examples *Salmonella typhimurium* and *Serratia marcesans*, utilizing plasmids that can replicate and express heterologous gene sequences therein. Yeast, such as *Saccharomyces cerevisiae*, may also be employed to advantage as host organism in the preparation of the insertion proteins hereof by expression of genes coding therefor under the control of a yeast promoter. (See the copending U.S. patent application of Hitzeman *et al.*, filed February 25, 1981 (Attorney Docket No. 100/43), assignee Genentech, Inc. *et al.*, or the corresponding European Application 82300949.3 which are incorporated herein by reference.

Preferred embodiments of the invention will now be described with reference to the accompanying drawings in which:

Figs. 1A and B are diagrams for use in explaining the construction of plasmid pHSA1;

Fig. 2 shows the immunoprecipitation of bacterially synthesised HSA; and

Fig. 3 shows the amino acid sequence of HSA and the corresponding DNA sequence.

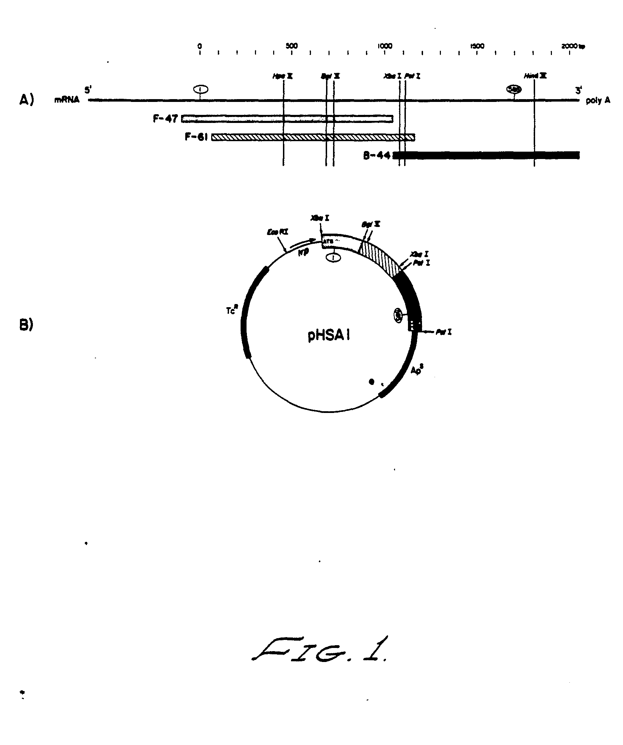

In Fig. 1A, the top line represents the mRNA coding for the human serum albumin protein and below it the regions contained in the cDNA clones F-47, F-61 and B-44 described further herein. The initial and final amino acid codons of the mature HSA mRNA are indicated by circled 1 and 585 respectively. Restriction enconuclease sites involved in the construction of pHSA1 are shown by vertical lines. An approximate size scale is nucleotides is included.

The completed plasmid pHSA1 is shown in Fig. 1B, with HSA coding regions derived from cDNA clones shaded as in A). Selected restriction sites and terminal codons number 1 and 585 are indicated as above. The *E. coli* trp promoter-operator region is shown with an arrow representing the direction of transcription. G:C denotes an oligo dG:dC tail. The leftmost XbaI site and the initiation codon ATG were added synthetically. The tetracycline (Tc) and ampicillin (Ap) resistance genes in the pBR322 portion of pHSA1 are indicated by a heavy line.

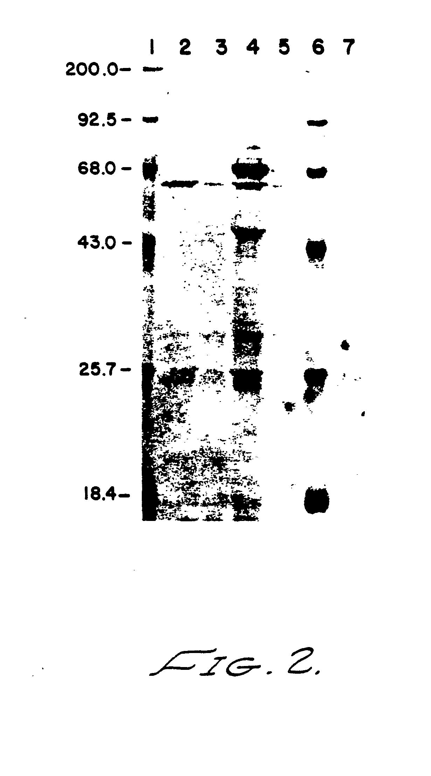

Figure 2 depicts the immunoprecipitation of bacterially synthesized HSA.

*E. coli* cells transformed with albumin expression plasmid pHSA1 (lanes 4 and 5) or control

plasmid pLeIFA25 (containing an interferon α gene in the identical expression vehicle; lanes 2, 3 and 7) were grown in $^{35}$S-methionine-supplemented media. Samples in lanes 2, 4 and 7 were induced for expression from the trp promoter in M9 media lacking tryptophan; samples in lanes 3 and 5 were grown in tryptophan-containing LB broth to repress the trp promoter. Each sample lane of the autoradiograph of the SDS-polyacrylamide gel presented here contains labeled protein immunoprecipitated from 0.75 ml of cells at a density of $A_{550}=1$. Lanes 1 and 6 contain radioactive protein standards (BRL) whose molecular weight in kilodaltons is indicated at the left. Bacterially synthesized HSA is seen in lane 4 comigrating with the 68,000 d $^{14}$C-labeled bovine serum albumin standards. Increased production of serum albumin in the induced versus repressed culture of pHSA1 represents higher levels of synthesis of plasmid encoded protein rather than a difference in $^{35}$S-methionine pool specific activities for minimal versus rich media (data not shown). The sharp band at 60,000 d is an apparent artifact; this band is seen in both induced and repressed pHSA1 and control transformants, and binds to preimmune (lane 7) as well as anti-HSA IgGs (lanes 2—5). The minor 47,000 d band in lane 4 is apparently plasmid encoded and may represent a prematurely terminated form of bacterially synthesized HSA.

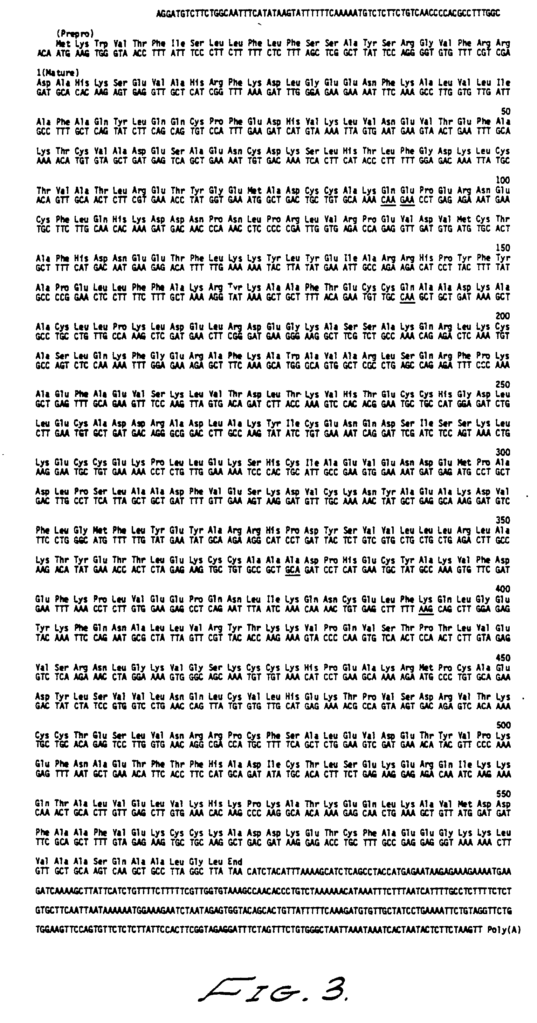

Figure 3 depicts the nucleotide and amino acid sequence of human serum albumin.

The DNA sequence of the mature protein coding and 3′ untranslated regions of HSA mRNA were determined from the recombinant plasmid pHSA1. The DNA sequence of the prepro peptide coding and 5′ untranslated regions were determined from the plasmid P-14 (see text). Predicted amino acids are included above the DNA sequence and are numbered from the first residue of the mature protein. The preceding 24 amino acids comprise the prepro peptide. The five amino acid residues which disagree with the protein sequence of HSA reported by both Dayhoff (9) and Moulon et al. (12) are underlined. The above nucleotide sequence probably does not extend to the true 5′ terminus of HSA mRNA. In the albumin direct expression plasmid pHSA1, the mature protein coding region is immediately preceded by the E. coli trp promoter-operator-leader peptide ribosome binding site (36, 37), an artificial Xbal site, and an artificial initiation codon ATG; the prepro region has been excised. The nucleotides preceding HSA codon No. 1 in pHSA1 read

5′-TCACGTAAAAAGGGTATCTAGATG.

Detailed description
(A) Synthesis and cloning of cDNA.

Poly(A)+ RNA was prepared from quickly frozen human liver samples obtained by biopsy or from cadaver donors by either ribonucleoside-vanadyl complex (17) or guanidinium thiocyanate (18) procedures. cDNA reactions were performed essentially as described in (19) employing as primers either oligo-deoxynucleotides prepared by the phosphotriester method (20) or oligo $(dT)_{12-18}$ (Collaborative Research). For typical cDNA reactions 25—35 µg of poly(A)+ RNA and 40—80 pmol of oligonucleotide primer were heated at 90° for 5 minutes in 50 mM NaCl. The reaction mixture was brought to final concentrations of 20 mM Tris HCl pH 8.3, 20 mM KCl, 8 mM $MgCl_2$, 30 mM dithiothreitol, 1 mM dATP, dCTP, dGTP, dTTP (plus $^{32}$P-dCTP (Amersham) to follow recovery of product) and allowed to anneal at 42°C for 5′. 100 units of AMV reverse transcriptase (BRL) were added and incubation continued at 42° for 45 minutes. Second strand DNA synthesis, SI treatment, size selection on polyacrylamide gels, deoxy (C) tailing and annealing to pBR322 which was cleaved with PstI and deoxy (G) tailed, were performed as previously described (21, 22). The annealed mixture was used to transform E. coli K-12 strain 294 (23) by a published procedure (24).

(B) Screening of recombinant plasmids with $^{32}$P-labelled Probes.

E. coli transformants were grown on LB-agar plates containing 5 µg/ml tetracycline, transferred to nitrocellulose filter paper (Schleicher and Schuell, BA85) and tested by hybridization using a modification of the in situ colony screening procedure (25). $^{32}$P-end labelled (26) oligodeoxynucleotide fragments of from 12 to 16 nucleotides in length were used as direct hybridization probes, or $^{32}$P-cDNA probes were synthesized from RNA using oligo (dT) or oligodeoxynucleotide primers (19). Filters were hybridized overnight in 5X Denhardt's solution (27), 5×SSC, (1×SSC=1.5M NaCl, 0.15 M Na Citrate) 50 mM Na phosphate pH 6.8, 20 µg/ml salmon sperm DNA at a temperatures ranging from 4° to 42° and washed in salt concentrations varying from 1 to 0.2×SSC plus 0.1 percent SDS at temperatures ranging from 4° to 42° depending on the length of the $^{32}$P-labelled probe (28). Dried filters were exposed to Kodak XR-2 X-ray film using DuPont Lightning-Plus intensifying screens at −80°.

(C) DNA preparation and restriction enzyme analysis.

Plasmid DNA was prepared in either large scale (29) or small scale ("miniprep"; 30) quantities and cleaved by restriction endonucleases (New England Biolabs, BRL) following manufacturers conditions. Slab gel electrophoresis conditions and electroelution of DNA fragments from gels have been described (31).

(D) DNA sequencing.

DNA sequencing was accomplished by both the method of Maxam and Gilbert (26) utilizing end-labelled DNA fragments and by dideoxy chain termination (32) on single stranded DNA from phage M13 mP7 subclones (33) utilizing synthetic oligonucleotide (20) primers. Each region was independently sequenced several times.

(E) Construction of 5' end of ablumin gene for direct expression of HSA.

10 µg (~16 pmol) of the ~1200 bp PstI insert of plasmid F-47 was boiled in $H_2O$ for 5 minutes and combined with 100 pmol of $^{32}$P-end labelled 5' primer (dATGGATGCACACAAG). The mixture was quenched on ice and brought to a final volume of 120 µl of 6 mM Tris HCl pH 7.5, 6 mM $MgCl_2$, 60 mM NaCl, 0.5 mM dATP, dCTP, dGTP, dTTP at 0°. 10 units of DNA polymerase I Klenow fragment (Boehringer-Mannheim) were added and the mixture incubated at 24° for 5 hr. Following phenol/chloroform extraction, the product was digested with HpaII, electrophoresed in a 5 percent polyacrylamide gel, and the desired 450 bp fragment electroeluted. the single stranded overhang produced by XbaI digestion of the vector plasmid pLeIF A25 (21) was filled in to produce blunt DNA ends by adding deoxynucleoside triphosphates to 10 µM and 10 units DNA polymerase I Klenow fragment to the restriction endonuclease reaction mix and incubating at 12° for 10 minutes. Restriction endonuclease fragments (0.1—1 µg in approximate molar equality) were annealed and ligated overnight at 12° in 20 µl of 50 mM Tris HCl pH 7.6, 10 mM $MgCl_2$, 0.1 mM EDTA, 5 mM dithiothreitol, 1 mM rATP with 50 units T4 ligase (N.E. Biolabs). Further details of plasmid construction are discussed below.

(F) Protein analysis.

Two ml cultures of recombinant *E. coli* strains were grown in either LB or M9 media plus 5 µg/ml tetracycline to densities of $A_{550}=1.0$, pelleted, washed, repelleted, and suspended in 2 ml of LB or supplemented M9 (M9+0.2 percent glucose, 1 µg/ml thiamine, 20 µg/ml standard amino acids except methionine was 2 µg/ml and tryptophan was excluded). Each growth medium also contained 5 µg/ml tetracycline and 100 µCi $^{35}$S-methionine (NEN; 1200 Ci/mmol). After 1 hr incubation at 37°, bacteria was pelleted, freeze-thawed and resuspended in 200 µl 50 mM Tris HCl pH 7.5, 0.12 mM NaEDTA then placed on ice for 10 minutes following subsequent additions of lysozyme to 1 mg/ml, NP40 0.2 percent, and NaCl to 0.35 M. The lysate was adjusted to 10 mM $MgCl_2$ and incubated with 50 µg/ml DNase I (Worthington) on ice for 30 min. Insoluble material was removed by mild centrifugation. Samples were immunoprecipitated with rabbit anti-HSA (Cappel Labs) and staphylococcal absorbent (Pansorbin; Cal Biochem) as described (34), and subjected to SDS polyacrylamide gel electrophoresis (35).

(G) cDNA cloning.

Initial cDNA clones primed with aligo (dT) were screened by colony hybridization with both total liver cDNA (to identify abundant RNA species containing clones) and with two $^{32}$P-labelled cDNAs primed from liver mRNA by two sets of four 11 base oligodeoxynucleotides synthesized to represent the possible coding variations for amino acids 546—549 and 294—297 of HSA.

Positive colonies never contained more than about the 3' 1/2 of the protein coding region of the expected HSA mRNA sequence. (The longest of these recombinants was designated B-44). Since existing procedures were unable to directly copy an mRNA of the expected size (~2000 bp), synthetic oligodeoxynucleotides were prepared to correspond to the antimessage strand at regions near the 5' extreme of B-44. From the nucleotide sequence of B-44, we constructed a 12 base oligodeoxynucleotide corresponding to amino acids 369—373. This was used to prime cDNA synthesis of liver mRNA and produce cDNA clones in pBR322 containing the 5' portion of the HSA message while overlapping the existing B-44 recombinant. Approximately 400 resulting clones were screened by colony hybridization with a 16 base oligodeoxynucleotide fragment located slightly upstream in the mRNA sequence we had thus far determined. Approximately 40 percent of the colonies hybridized to both probes. Many of those colonies which failed to contain hybridizing plasmids presumably resulted from RNA self-priming or priming with contaminating oligo (dT) during reverse transcription, or lost the 3' region containing the sequence used for screening. "Miniprep" amounts of plasmid DNA from hybridizing colonies were digested with PstI. Three recombinant plasmids contained sufficiently large inserts to code for the remaining 5' portion of the HSA message. Two of these (F-15 and F-47) contained the extreme 5' coding portion of the gene but failed to extend back to a PstI site necessary for joining with B-44 to reform the complete albumin gene. Recombinant F-61 possessed this site but lacked the 5' extreme end. A three part reconstruction of the entire message sequence was possible employing restriction endonuclease sites in common with the part length clones F-47, F-61 and B-44 (Fig. 1).

An additional cDNA clone extending further 5' was obtained by similar oligodeoxynucleotide primed cDNA synthesis (from a primer corresponding to amino acid codon No. 175—179). Although not employed in the construction of the mature HSA expression plasmid, this cDNA clone (P-14) allowed determination of the DNA sequence of the "prepro" peptide coding and 5' non-coding regions of the HSA mRNA.

The mature HSA mRNA sequence was joined to a vector plasmid for direct expression of the mature protein in *E. coli* via the trp promoter-operator. The plasmid pLeIF A25 directs the expression of human leukocyte interferon A (IFNα2) (21). It was digested with XbaI and the cleavage site "filled in" to produce blunt DNA ends with DNA polymerase I Klenow fragment and deoxynucleoside triphosphates. After subsequent digestion with PstI, a "vector" fragment was gel purified that contained pBR322 sequences and a 300 bp fragment of the *E. coli* trp promoter, operator, and ribosome binding site of the trp leader peptide terminating in the artificially blunt ended XbaI cleavage site. A 15 base oligodeoxynucleotide was designed to contain the initiation codon ATG followed by the 12 nucleotides coding for the first four amino acids of HSA as determined by DNA sequence analysis of clone F-47. In a process referred to as "primer repair", the gene-containing Pstl fragment F-47 was denatured, annealed with excess 15-mer and reacted with DNA polymerase I Klenow fragment and deoxynucleoside triphosphates. This reaction extends a new second strand downstream from the annealed oligonucleotide, degrades the single stranded DNA upstream of codon number one and then polymerizes upstream three nucleotides complementary to ATG. In addition, when this product is blunt-end ligated to the prepared vector fragment, its initial adenosine residue recreates an Xbal restriction site. Following the primer repair reaction, the DNA was digested with Hpall and a 450 bp fragment containing the 5' portion of the mature albumin gene was gel purified (see Fig. 1). This fragment was annealed and ligated to the vector fragment and to the gel isolated Hpall to Pstl portion of F-47 and used to transform E. coli cells. Diagnostic restriction endonuclease digests of plasmid minipreps identified the recombinant A-26 which contained the 5' portion of the mature albumin coding region ligated properly to the trp promoter-operator. For the final steps in assembly, the A-26 plasmid was digested with BgPll plus Pstl and the ~4 kb fragment was gel purified. This was annealed and ligated to a 390 bp Pstl, Bglll partial digestion fragment purified from F-61 and a 1000 bp Pstl fragment of B-44. Restriction endonuclease analysis of resulting transformants identified plasmids containing the entire HSA coding sequence properly aligned for direct expression of the mature protein. One such recombinant plasmid was designated pHSA1. When E. coli containing pHSA1 is grown in minimal media lacking tryptophan, the cells produce a protein which specifically reacts with HSA antibodies and comigrates with HSA in SDS polyacrylamide electrophoresis (Fig. 2). No such protein is produced by identical recombinants grown in rich broth, implying that production in E. coli of the putative HSA protein is under control of the trp promoter-operator as designed. To insure the integrity of the HSA structural gene in the recombinant plasmid, pHSA1 was subject to DNA sequence analysis.

(H) DNA sequence analysis

The albumin cDNA portion (and surrounding regions) of pHSA1 were sequenced to completion by both the chemical degradation method of Maxam and Giblert (26) and the dideoxy chain termination procedure employing templates derived from single stranded M13 mP7 phage derivatives (32, 33). All nucleotides were sequenced at least twice. The DNA sequence is shown in Fig. 3 along with the predicted amino acid sequence of the HSA protein. The DNA sequence farther 5' to the mature HSA coding region was also determined from the cDNA clone P-14 and is included in Fig. 3.

DNA sequence analysis confirmed that the artificial initiation codon and the complete mature HSA coding sequence directly follows the E. coli trp promoter-operator as desired. The ATG initiator follows the putative E. coli ribosome binding sequence (36) of the trp leader peptide (37) by 9 nucleotides.

Translation of the DNA sequence of pHSA1 predicts a mature HSA protein of 585 amino acids. Various published protein sequences of HSA disagree at about 20 amino acids. The present sequence differs by eleven residues from Moulon et al. (12), and by 28 residues from that reported in the Dayhoff catalogue (9) credited as arising primarily from Behrens et al. (10) with contributions by Moulon et al. (12). Most of these differences represent inversions of pairs of adjacent residues or glutamine-glutamic acid disagreements. Only at five of the 585 residues does our sequence differ from the residue reported by both Dayhoff (9) and Moulon et al. (12), and three of these five differences represent glutamine-glutamic acid interchanges (underlined in Figure 3). At all discrepant positions the nucleotide sequencing has been carefully rechecked and it is unlikely that DNA sequencing errors are the cause of these reported differences. The possibility of artifacts introduced by cDNA cloning cannot be ruled out. However, other likely explanations exist for the amino acid sequence differences among various reports. These include changes in amidation (affecting glutamine-glutamic acid discrimination) occurring either in vivo or during protein sequencing (38). Polymorphism in HSA proteins may also account for some differences; over twenty genetic variants of HSA have been detected by protein electrophoresis (39) but have not yet been analyzed at the amino acid sequence level. It is also worth noting that our predicted HSA protein sequence is 585 amino acids long, in agreement with Moulon (12) but not Dayhoff (9). The difference is accounted for by the deletion (in ref. 9) of one phenylalanine (Phe) residue in a Phe-Phe pair at amino acids 156—157.

When compared to the DNA sequence of a rat serum albumin cDNA CLONE (16) the present mature HSA sequence shares 74 percent homology at the nucleotide and 73 percent homology at the amino acid level. (The rat SA protein is one amino acid shorter than HSA; the carboxy terminal residue of HSA is absent in the rat protein.) All 35 cysteine residues are located in identical positions in both proteins. The predicted "prepro" peptide region of HSA shares 76 percent nucleotide and 75 percent amino acid homology with that reported from the rat cDNA clone (16). Interspecies sequence homology is reduced in the portion of the 3' untranslated region which can be compared (the published rat cDNA clone ends before the 3' mRNA terminus). The HSA cDNA contains the hexanucleotide AATAAA 28 nucleotides before the site of poly (A) addition. This is a common feature of eukaryotic mRNAs first noted by Proudfoot and Brownlee (40).

## Pharmaceutical compositions

The compounds of the present invention can be formulated according to known methods to prepare pharmaceutically useful compositions, whereby the polypeptide hereof is combined in admixture with a pharmaceutically acceptable carrier vehicle. Suitable vehicles and their formulation are described in Remington's *Pharmaceutical Sciences* by E. W. Martin, which is hereby incorporated by reference. Such compositions will contain an effective amount of protein hereof together with a suitable amount of vehicle in order to prepare pharmaceutically acceptable compositions suitable for effective administration to the host. One preferred mode of administration is parenteral.

Bibliography
References

1. Rosenoer, V. M., Oratz, M., Rothschild, M.A. eds. (1977) Albumin Structure, Function and Uses, Pergammon Press, Oxford.
2. Peters, T. (1977) Clin. Chem. (Winston-Salem, N.C. (23, 5—12.
3. Ruoslahti, E. and Terry W. D. (1976) Nature 260, 804—805.
4. Sala-Trepat, J. M., Dever, J., Sargent, T. D., Thomas, K., Sell, S. and Bonner, J. (1979) Biochemistry 18, 2167—2178.
5. Jagodzinski, L. L., Sargent, T. D., Yang, M., Glackin, C. and Bonner, J. (1981) Proc. Natl. Acad. Sci. USA 78, 3521—3525.
6. Ruoslahti, E. and Terry, W. D. (1976) Nature 260, 804—805.
7. Yachnin, S., Hsu, R., Heinrikson, R. L. and Miller, J. B. (1977) Biochim. Biophys. Acta 493, 418—428.
8. Aoyagi, Y., Ikenaka, T. and Ichida, F. (1977) Cancer Research 37, 3663—3667.
9. Dayhoff, M. (1978) Atlas of Protein Sequence and Structure, Vol. 5, Suppl. 3, p. 266, National Biomedical Research Foundation, Washington.
10. Behrens, P. Q., Spiekerman, A. M. and Brown J. R. (1975) Fed. Proc. 34, 591.
11. Brown, J. R. (1977) in Resoneor *et al.* (1977), pp. 27—52.
12. Meloun, B., Moravek, L. and Kostka, V. (1975) Febs Letters 58, 134—137.
13. Judah, J. O., Gamble M., and Steadman, J. H. (1973) Biochem J. 134, 1083—1091.
14. Russell, J. H. and Geller, D. M. (1973) Biochem Biophys. Res. Commun. 55, 239—245.
15. MacGillivray, R. T., Chung, D. W. and Davie, E. W. (1979) Eur. J. Biochem.*98* 477—485.
16. Sargent, T. D., Yang, M. and Bonner, J. (1981) Proc. Natl. Acad. Sci. USA 78, 243—246.
17. Berger, S. L. and Birkenmeier, C. S. (1979) Biochemistry 18, 5143—5149.
18. Ullrich A., Shine, J., Chirgwin, R., Pictet, R., Tischer, E., Rutter, W. J. and Goodman, H.M. (1977) Science 196, 1313—1315.
19. Goeddel, D. V., Yelverton, E., Ullrich, A., Heyneker, H.L., Miozzari, G., Holmes, W., Seeburg, P. H., Dull, T., May, L., Stebbing, N., Crea, R., Maeda, S., McCandliss, R., Sloma, A., Tabor, J. M., Gross, M., Familletti, P. C. and Pestka, S. (1980) Nature 287, 411—416.
20. Crea, R. and Horn, T. (1980) Nucleic Acids Res. 8, 2331—2348.
21. Goeddel, D. V., Heyneker, H. L., Hozumi, T., Arentzen, R., Itakura, K., Yansura, D. G., Ross, M. J., Niozzari, G., Crea, R. and Seeburg, P. H. (1979) Nature 281, 544—548.
22. Goeddel, D. V, Shepard, H. M., Yelverton, E., Leung, D. and Crea, R. (1980) Nucleic Acids Res. 8, 4057—4074.
23. Backman, K., Ptashne, M. and Gilbert, W. (1976) Proc. Natl. Acad. Sci, USA 73, 4174—4178.
24. Hershfield, V., Boyer, H. W., Yanofsky, C., Lovett, M. A. and Helinski, D. R. (1974) Proc. Natl. Acad. Sci. USA 71, 3455—3459.
25. Grunstein, M., and Hogness, D. S. (1975) Proc. Natl. Acad. Sci. USA 3961—3965.
26. Maxam, A. M. and Gilbert, W. (1980) Methods Enzymol. 65, 499—560.
27. Denhardt, D. T. (1966) Biochem, Biophys. Res. Commun. 23, 461—467.
28. Wallace, R. B., Johnson, M. J. Hirose, T., Miyake, T., Kawashima, E. H. and Itakura. K. (1981) Nucleic Acids Research 9, 879—893.
29. Blin, N. Stafford, D. W. (1976) Nucleic Acids Res. 3, 2303—2308.
30. Birnboim H. C. and Doly, J. (1979) Nucleic Acids Research 7, 1513—1523.
31. Lawn, R. M., Adelman, J., Franke, A. E., Houck, C. M., Gross, M., Najarian, R. and Goeddel, D. V. (1981) Nucleic Acids Research 9, 1045—1052.
32. Sanger, F., Nicklen, S. and Coulson, A. R. (1977) Proc. Natl. Acad. Sci. USA 74, 5463—5467.
33. Messing, J. Crea, R. and Seeburg, P. H. (1981) Nucleic Acids Res. *9* 309—322.
34. Kessler, S. W. (1976) J. Immunology 117, 1482—1490.
35. Laemmli. U.K. (1970) Nature 277, 680—685.
36. Shine, J. and Dalgarno L. (1974) Proc. Natl. Acad. Sci. USA 71, 1342—1346.
37. Platt, T., Squires, C. and Yanofsky, C. (1976) J. Mol. Biol. 103, 411—420.
38. Robinson, A. B. and Rudd, C. J. (1974) Current Topics in Cellular Regulation, 247—295.
39. Weitkamp, L. R., Salzano F. M., Neel, J. V., Porta, F., Geerdink, R. A. and Tarnoky, A. L. (1973) Ann, Hum. Genet., Lond. 36. 381—391.
40. Proudfoot, N. J. and Brownlee, G. G. (1976) Nature 263, 211—214.
41. Gorin, M. B., Cooper, D. L. Eiferman, F., Van de Rijn P. and Tilghman, S. M. (1981) J. Mol. Biol. 256, 1954—1959.
42. Kiousois, D., Eiferman, F., Van de Rijn, P., Gorin, M. B., Ingram, R. S. and Tilghman, S. M. (1981) J. Mol. Biol. 256, 1960—1967.

**Claims for the Contracting States: BE, CH, DE, FR, GB, IT, LI, LU, NL SE**

1. A DNA isolate comprising a continuous sequence encoding human serum albumin of the amino acid sequence depicted in Fig. 3 hereof and genetic variants thereof.

2. An expression vehicle comprising a DNA coding sequence according to claim 1 operably linked with a DNA vector capable of effecting the microbial expression of said sequence so as to prepare the corresponding human serum albumin.

3. A microorganism transformed with the vehicle according to claim 2.

4. The microorganism according to claim 3, obtained by transforming an *E. coli* bacterial or a yeast strain.

5. A process which comprises microbially expressing human serum albumin of the amino acid sequence depicted in Fig. 3 hereof and genetic variants thereof.

6. A process which comprises expressing human serum albumin in a transformed microorganism of claim 3 or claim 4.

7. A process of claim 5 or claim 6 which further includes using the human serum albumin to prepare a pharmaceutical composition for the therapeutic treatment of humans.

**Claims for the Contracting State: AT**

1. A method which comprises the preparation of a DNA isolate comprising a continuous sequence encoding human serum albumin of the amino acid sequence depicted in Fig. 3 hereof and genetic variants thereof.

2. A method which comprises the preparation of an expression vehicle comprising a DNA coding sequence according to claim 1 operably linked with a DNA vector capable of effecting the microbial expression of said sequence so as to prepare the corresponding human serum albumin.

3. A microorganism transformed with the expression vehicle as defined in claim 2.

4. The microorganism according to claim 3, obtained by transforming an *E. coli* bacterial or a yeast strain.

5. A process which comprises microbially expressing human serum albumin of the amino acid sequence depicted in Fig. 3 hereof and genetic variants thereof.

6. A process which comprises expressing human serum albumin in a transformed microorganism of claim 3 or claim 4.

7. A process of claim 4 or claim 6 which further includes using the human serum albumin to prepare a pharmaceutical composition for the therapeutic treatment of humans.

**Patentansprüche für die Vertragsstaaten: BE CH DE FR GB IT LI LU NL SE**

1. DNA-Isolat umfassend eine kontinuierliche Sequenz kodierend für menschliches Serum-Albumin der in Fig. 3 der vorliegenden Anmeldung dargestellten Aminosäuresequenz und genetische Varianten desselben.

2. Expressionsvehikel umfassend eine DNA-Kodierungssequenz nach Anspruch 1, die betriebsfähig an einen DNA-Vektor gebunden ist, der fähig ist, die mikrobielle Expression der genannten Sequenz zu bewirken, um das entsprechende menschliche Serum-Albumin herzustellen.

3. Mikroorganismus, der mit den Vehikel nach Anspruch 2 transformiert ist.

4. Mikroorganismus nach Anspruch 3, der durch Transformieren eines E. coli Bakterien- oder eines Hefestammes erhalten wird.

5. Verfahren umfassend die mikrobielle Expression von menschlichem Serum-Albumin mit der in Fig. 3 der vorliegenden Anmeldung dargestellten Aminosäuresequenz und genetische Varianten desselben.

6. Verfahren umfassend die Expression von menschlichem Serum-Albumin in einem transformierten Mikroorganismus nach Anspruch 3 oder 4.

7. Verfahren nach Anspruch 5 oder 6, das weiters die Verwendung des menschlichen Serum-Albumins zur Herstellung einer pharmazeutischen Zusammensetzung für die therapeutische Behandlung des Menschen umfaßt.

**Patentansprüche für den Vertagsstaat: AT**

1. Verfahren umfassend die Herstellung eines DNA-Isolates, das eine kontinuierliche Sequenz kodierend für menschliches Serum-Albumin der in Fig. 3 der vorliegenden Anmeldung dargestellten Aminosäuresequenz und genetische Varianten desselben umfaßt.

2. Verfahren umfassend die Herstellung eines Expressionsvehikels, das eine für DNA kodierende Sequenz nach Anspruch 1 umfaßt, die betriebsfähig mit einem DNA-Vektor verbunden ist, der fähig ist, die mikrobielle Expression der genannten Sequenz zu bewirken, um das entsprechende menschliche Serum-Albumin herzustellen.

3. Mikroorganismus, der mit dem in Anspruch 2 definierten Expressionsvehikel transformiert ist.

4. Mikroorganismus nach Anspruch 3, der durch Transformieren eines E. coli-Bakterien- oder eines Hefestammes erhalten wird.

5. Verfahren umfassend die mikrobielle Expression von menschlichem Serum-Albumin der in Fig. 3 der vorliegenden Anmeldung dargestellten Aminosäuresequenz und genetischer Varianten desselben.

6. Verfahren umfassend die Expression von menschlichem Serum-Albumin in einem transformierten Mikroorganismus nach Anspruch 3 oder 4.

7. Verfahren nach Anspruch 5 oder 6, weiters umfassend die Verwendung des menschlichen Serum-Albumins zur Herstellung einer pharmazeutischen Zusammensetzung für die therapeutische Behandlung des Menschen.

**Revendications pour les Etats contractants: BE CH DE FR GB IT LI LU NL SE**

1. Produit isolé d'ADN comprenant une séquence continue codant pour la sérum-albumine humaine de la séquence d'acides aminés présentée à la figure 3 et ses variants génétiques.

2. Véhicule d'expression comprenant une séquence d'ADN de codage selon la revendication 1 lié de façon opératoire avec un vecteur d'ADN capable d'accomplir l'expression microbiologique de ladite séquence de façon à préparer la sérum-albumine humaine correspondante.

3. Micro-organisme transformé avec la véhicule selon la revendication 2.

4. Micro-organisme selon la revendication 3, obtenu par transformation d'une souche bactérienne de *E. coli* ou d'une souche de levure.

5. Procédé qui comprend l'expression microbiologique de la sérum-albumine humaine de la séquence d'acides aminés présentée à la figure 3 et de ses variants génétiques.

6. Procédé qui comprend l'expression de la sérum-albumine humaine dans un micro-organisme transformé selon la revendication 3 ou la revendication 4.

7. Procédé selon la revendication 5 ou la revendication 6, qui comprend en outre l'utilisation de la sérum-albumine humaine pour préparer une composition pharmaceutique pour le traitement thérapeutique des humains.

**Revendications pour l'Etat contractant: AT**

1. Procédé qui comprend la préparation d'un produit isolé d'ADN comprenant une séquence continue codant pour la sérum-albumine humaine de la séquence d'acides aminés présentée à la figure 3 et ses variants génétiques.

2. Procédé qui comprend la préparation d'un véhicule d'expression comprenant une séquence d'ADN de codage selon la revendication 1 lié de façon opératoire à un vecteur d'ADN capable d'effectuer l'expression microbiologique de ladite séquence de façon à préparer la sérum-albumine correspondante.

3. Micro-organisme transformé avec le véhicule d'expression tel que défini à la revendication 2.

4. Micro-organism selon la revendication 3, obtenu par transformation d'une souche bactérienne de *E. coli* ou d'une souche de levure.

5. Procédé qui comprend l'expression microbiologique de la sérum-albumine humaine de la séquence d'acides aminés représentée à la figure 3 et de ses variants générique.

6. Procédé qui comprend l'expression de la sérum-albumine humaine dans un micro-organisme transformé selon la revendication 3 ou la revendication 4.

7. Procédé selon la revendication 5 ou la revendication 6 qui comprend en outre l'utilisation de la sérum-albumine humaine pour préparer une composition pharmaceutique pour le traitement thérapeutique des humains.

FIG.1.

*FIG. 2.*

# EP 0 073 646 B1

AGGATGTCTTCTGGCAATTTCATATAAGTATTTTTTCAAAAATGTCTCTTCTGTCAACCCCACGCCTTTGGC

(Prepro)  
Met Lys Trp Val Thr Phe Ile Ser Leu Leu Phe Leu Phe Ser Ser Ala Tyr Ser Arg Gly Val Phe Arg Arg  
ACA ATG AAG TGG GTA ACC TTT ATT TCC CTT CTT TTT CTC TTT AGC TCG GCT TAT TCC AGG GGT GTG TTT CGT CGA

1(Mature)  
Asp Ala His Lys Ser Glu Val Ala His Arg Phe Lys Asp Leu Gly Glu Glu Asn Phe Lys Ala Leu Val Leu Ile  
GAT GCA CAC AAG AGT GAG GTT GCT CAT CGG TTT AAA GAT TTG GGA GAA GAA AAT TTC AAA GCC TTG GTG TTG ATT

50  
Ala Phe Ala Gln Tyr Leu Gln Gln Cys Pro Phe Glu Asp His Val Lys Leu Val Asn Glu Val Thr Glu Phe Ala  
GCC TTT GCT CAG TAT CTT CAG CAG TGT CCA TTT GAA GAT CAT GTA AAA TTA GTG AAT GAA GTA ACT GAA TTT GCA

Lys Thr Cys Val Ala Asp Glu Ser Ala Glu Asn Cys Asp Lys Ser Leu His Thr Leu Phe Gly Asp Lys Leu Cys  
AAA ACA TGT GTA GCT GAT GAG TCA GCT GAA AAT TGT GAC AAA TCA CTT CAT ACC CTT TTT GGA GAC AAA TTA TGC

100  
Thr Val Ala Thr Leu Arg Glu Thr Tyr Gly Glu Met Ala Asp Cys Cys Ala Lys Gln Glu Pro Glu Arg Asn Glu  
ACA GTT GCA ACT CTT CGT GAA ACC TAT GGT GAA ATG GCT GAC TGC TGT GCA AAA CAA GAA CCT GAG AGA AAT GAA

Cys Phe Leu Gln His Lys Asp Asp Asn Pro Asn Leu Pro Arg Leu Val Arg Pro Glu Val Asp Val Met Cys Thr  
TGC TTC TTG CAA CAC AAA GAT GAC AAC CCA AAC CTC CCC CGA TTG GTG AGA CCA GAG GTT GAT GTG ATG TGC ACT

150  
Ala Phe His Asp Asn Glu Glu Thr Phe Leu Lys Lys Tyr Leu Tyr Glu Ile Ala Arg Arg His Pro Tyr Phe Tyr  
GCT TTT CAT GAC AAT GAA GAG ACA TTT TTG AAA AAA TAC TTA TAT GAA ATT GCC AGA AGA CAT CCT TAC TTT TAT

Ala Pro Glu Leu Leu Phe Phe Ala Lys Arg Tyr Lys Ala Ala Phe Thr Glu Cys Cys Gln Ala Ala Asp Lys Ala  
GCC CCG GAA CTC CTT TTC TTT GCT AAA AGG TAT AAA GCT GCT TTT ACA GAA TGT TGC CAA GCT GCT GAT AAA GCT

200  
Ala Cys Leu Leu Pro Lys Leu Asp Glu Leu Arg Asp Glu Gly Lys Ala Ser Ser Ala Lys Gln Arg Leu Lys Cys  
GCC TGC CTG TTG CCA AAG CTC GAT GAA CTT CGG GAT GAA GGG AAG GCT TCG TCT GCC AAA CAG AGA CTC AAA TGT

Ala Ser Leu Gln Lys Phe Gly Glu Arg Ala Phe Lys Ala Trp Ala Val Ala Arg Leu Ser Gln Arg Phe Pro Lys  
GCC AGT CTC CAA AAA TTT GGA GAA AGA GCT TTC AAA GCA TGG GCA GTG GCT CGC CTG AGC CAG AGA TTT CCC AAA

250  
Ala Glu Phe Ala Glu Val Ser Lys Leu Val Thr Asp Leu Thr Lys Val His Thr Glu Cys Cys His Gly Asp Leu  
GCT GAG TTT GCA GAA GTT TCC AAG TTA GTG ACA GAT CTT ACC AAA GTC CAC ACG GAA TGC TGC CAT GGA GAT CTG

Leu Glu Cys Ala Asp Asp Arg Ala Asp Leu Ala Lys Tyr Ile Cys Glu Asn Gln Asp Ser Ile Ser Ser Lys Leu  
CTT GAA TGT GCT GAT GAC AGG GCG GAC CTT GCC AAG TAT ATC TGT GAA AAT CAG GAT TCG ATC TCC AGT AAA CTG

300  
Lys Glu Cys Cys Glu Lys Pro Leu Leu Glu Lys Ser His Cys Ile Ala Glu Val Glu Asn Asp Glu Met Pro Ala  
AAG GAA TGC TGT GAA AAA CCT CTG TTG GAA AAA TCC CAC TGC ATT GCC GAA GTG GAA AAT GAT GAG ATG CCT GCT

Asp Leu Pro Ser Leu Ala Ala Asp Phe Val Glu Ser Lys Asp Val Cys Lys Asn Tyr Ala Glu Ala Lys Asp Val  
GAC TTG CCT TCA TTA GCT GCT GAT TTT GTT GAA AGT AAG GAT GTT TGC AAA AAC TAT GCT GAG GCA AAG GAT GTC

350  
Phe Leu Gly Met Phe Leu Tyr Glu Tyr Ala Arg Arg His Pro Asp Tyr Ser Val Val Leu Leu Leu Arg Leu Ala  
TTC CTG GGC ATG TTT TTG TAT GAA TAT GCA AGA AGG CAT CCT GAT TAC TCT GTC GTG CTG CTG CTG AGA CTT GCC

Lys Thr Tyr Glu Thr Thr Leu Glu Lys Cys Cys Ala Ala Ala Asp Pro His Glu Cys Tyr Ala Lys Val Phe Asp  
AAG ACA TAT GAA ACC ACT CTA GAG AAG TGC TGT GCC GCT GCA GAT CCT CAT GAA TGC TAT GCC AAA GTG TTC GAT

400  
Glu Phe Lys Pro Leu Val Glu Glu Pro Gln Asn Leu Ile Lys Gln Asn Cys Glu Leu Phe Lys Gln Leu Gly Glu  
GAA TTT AAA CCT CTT GTG GAA GAG CCT CAG AAT TTA ATC AAA CAA AAC TGT GAG CTT TTT AAG CAG CTT GGA GAG

Tyr Lys Phe Gln Asn Ala Leu Leu Val Arg Tyr Thr Lys Lys Val Pro Gln Val Ser Thr Pro Thr Leu Val Glu  
TAC AAA TTC CAG AAT GCG CTA TTA GTT CGT TAC ACC AAG AAA GTA CCC CAA GTG TCA ACT CCA ACT CTT GTA GAG

450  
Val Ser Arg Asn Leu Gly Lys Val Gly Ser Lys Cys Cys Lys His Pro Glu Ala Lys Arg Met Pro Cys Ala Glu  
GTC TCA AGA AAC CTA GGA AAA GTG GGC AGC AAA TGT TGT AAA CAT CCT GAA GCA AAA AGA ATG CCC TGT GCA GAA

Asp Tyr Leu Ser Val Val Leu Asn Gln Leu Cys Val Leu His Glu Lys Thr Pro Val Ser Asp Arg Val Thr Lys  
GAC TAT CTA TCC GTG GTC CTG AAC CAG TTA TGT GTG TTG CAT GAG AAA ACG CCA GTA AGT GAC AGA GTC ACA AAA

500  
Cys Cys Thr Glu Ser Leu Val Asn Arg Arg Pro Cys Phe Ser Ala Leu Glu Val Asp Glu Thr Tyr Val Pro Lys  
TGC TGC ACA GAG TCC TTG GTG AAC AGG CGA CCA TGC TTT TCA GCT CTG GAA GTC GAT GAA ACA TAC GTT CCC AAA

Glu Phe Asn Ala Glu Thr Phe Thr Phe His Ala Asp Ile Cys Thr Leu Ser Glu Lys Glu Arg Gln Ile Lys Lys  
GAG TTT AAT GCT GAA ACA TTC ACC TTC CAT GCA GAT ATA TGC ACA CTT TCT GAG AAG GAG AGA CAA ATC AAG AAA

550  
Gln Thr Ala Leu Val Glu Leu Val Lys His Lys Pro Lys Ala Thr Lys Glu Gln Leu Lys Ala Val Met Asp Asp  
CAA ACT GCA CTT GTT GAG CTT GTG AAA CAC AAG CCC AAG GCA ACA AAA GAG CAA CTG AAA GCT GTT ATG GAT GAT

Phe Ala Ala Phe Val Glu Lys Cys Cys Lys Ala Asp Asp Lys Glu Thr Cys Phe Ala Glu Glu Gly Lys Lys Leu  
TTC GCA GCT TTT GTA GAG AAG TGC TGC AAG GCT GAC GAT AAG GAG ACC TGC TTT GCC GAG GAG GGT AAA AAA CTT

Val Ala Ala Ser Gln Ala Ala Leu Gly Leu End  
GTT GCT GCA AGT CAA GCT GCC TTA GGC TTA TAA CATCTACATTTAAAAGCATCTCAGCCTACCATGAGAATAAGAGAAAGAAAATGAA

GATCAAAAGCTTATTCATCTGTTTTCTTTTTCGTTGGTGTAAAGCCAACACCCTGTCTAAAAAACATAAATTTCTTTAATCATTTTGCCTCTTTTCTCT

GTGCTTCAATTAATAAAAAATGGAAAGAATCTAATAGAGTGGTACAGCACTGTTATTTTTCAAAGATGTGTTGCTATCCTGAAAATTCTGTAGGTTCTG

TGGAAGTTCCAGTGTTCTCTCTTATTCCACTTCGGTAGAGGATTTCTAGTTTCTGTGGGCTAATTAAATAAATCACTAATACTCTTCTAAGTT Poly(A)

$$\mathcal{FIG.\ 3.}$$

3